# FASCICULE DE BREVET EUROPEEN

(11) **EP 0 998 902 B1**
(45) Date de publication et mention de la délivrance du brevet: **04.04.2001**
(21) Numéro de dépôt: 99402409.9
(22) Date de dépôt: 01.10.1999
(51) Int. Cl.: A61K 7/00, A61K 7/48

(54) **Lotion cosmétique et/ou dermatologique poudrée et son utilisation**
Kosmetische und/oder dermatologische pulverartige Lotion und deren Verwendung
Cosmetic and/or dermatological powder lotion and its use

(30) Priorité: 04.11.1998 FR 9813867
(43) Date de publication de la demande: 10.05.2000
(73) Titulaire: L'OREAL, 75008 Paris (FR)
(72) Inventeur: Lebreton, Francoise, 91440 Bures/S/Yvette (FR)
(74) Mandataire: Rasson, Catherine

(56) Documents cités:
- EP-A- 0 447 286
- EP-A- 0 447 287
- EP-A- 0 486 394
- EP-A- 0 566 442
- EP-A- 0 692 237
- EP-A- 0 770 373
- GB-A- 2 238 242

## Description

L'invention a pour objet une lotion cosmétique et/ou dermatologique contenant une phase aqueuse et une phase poudrée, la phase poudrée comportant au moins une poudre active et des particules creuses à base d'au moins un polymère ou copolymère acrylique ou méthacrylique, et l'utilisation de la dite lotion pour le soin, le nettoyage, le démaquillage et/ou la coloration de la peau, des muqueuses et/ou du cuir chevelu.

Elle a aussi pour objet l'utilisation de particules creuses à base d'au moins un polymère ou copolymère acrylique ou méthacrylique dans une lotion cosmétique et/ou dermatologique contenant une phase aqueuse et une phase poudrée comportant au moins une poudre active, pour éviter le colmatage de la phase poudrée et conférer à la lotion de la douceur en application sur la peau, les muqueuses et/ou le cuir chevelu.

Le nettoyage de la peau est très important pour le soin du visage. Il doit être le plus performant possible car les résidus gras tels que l'excès de sébum, les restes des produits cosmétique et/ou dermatologiques utilisés quotidiennement et les produits de maquillage, notamment les produits "waterproof", résistants à l'eau, s'accumulent dans les replis cutanés et à la surface de la peau et peuvent obstruer les pores de la peau et entraîner l'apparition de boutons. Une mauvaise qualité de nettoyage ou de démaquillage est souvent responsable parmi d'autres facteurs de cause, d'un teint brouillé.

Dans le domaine du soin, du nettoyage et du démaquillage de la peau humaine, il est connu d'utiliser des lotions ou des toniques poudrés, c'est-à-dire comprenant deux phases : une phase aqueuse éventuellement additionnée d'alcool, et une phase constituée d'une poudre active, c'est-à-dire une poudre ayant une efficacité sur la peau, par exemple en éliminant le sébum ou en ayant un effet matifiant. Dans le flacon, ces deux phases sont séparées quand le produit est au repos et elles doivent être mélangées au moment de l'utilisation par agitation. Après quelques heures de repos, elles se séparent de nouveau en deux phases pour reprendre leur état initial.

Ainsi, le document JP9-48721 décrit une lotion contenant une phase hydro-éthanolique et une poudre poreuse ou absorbant l'eau telle que la poudre d'amidon ou la poudre d'anhydride silicique. Ces lotions permettent d'éliminer de la peau l'excès de sébum et d'absorber les salissures.

Toutefois, l'utilisation d'une lotion poudrée n'est pas toujours dépourvue d'inconvénients. En effet, la décantation après utilisation est souvent longue et incomplète, ce qui a pour conséquence un manque de limpidité de la phase aqueuse ou hydroalcoolique et donc un aspect rédhibitoire pour l'utilisateur. En outre, la phase poudrée a tendance à se colmater au fond du flacon et à former un "gâteau", ce qui rend souvent difficile la remise en suspension de la poudre lors de l'utilisation.

Pour limiter cet effet de colmatage, il est connu d'ajouter de la silice. Mais l'ajout de silice a pour inconvénient de donner à la lotion un caractère rêche désagréable pour l'utilisateur.

Il subsiste donc le besoin d'une lotion poudrée n'ayant pas les inconvénients de celles de l'art antérieur, et notamment ayant une phase poudrée qui ne se colmate pas, tout en étant douce à l'application.

La demanderesse a trouvé de manière surprenante que l'addition de particules creuses à base d'au moins un polymère ou copolymère acrylique ou méthacrylique, dans les lotions poudrées permet d'éviter le phénomène de colmatage tout en donnant une grande douceur à l'application à la lotion.

Aussi, l'invention a pour objet une lotion cosmétique et/ou dermatologique comprenant une phase aqueuse et une phase poudrée comportant au moins une poudre active, caractérisée en ce que !a phase poudrée comporte des particules creuses à base d'au moins un polymère ou copolymère acrylique ou méthacrylique, la phase aqueuse étant présente en une quantité allant de 90 à 99,99 % en poids par rapport au poids total de la lotion.

On entend par "phase aqueuse" dans la lotion selon l'invention la phase liquide de la lotion qu'elle soit strictement aqueuse ou hydroalcoolique, par opposition à la phase poudrée.

Cette lotion a l'avantage de présenter une phase poudrée qui ne se colmate pas et elle permet un traitement en douceur de la peau, des muqueuses et/ou du cuir chevelu.

L'invention a aussi pour objet l'utilisation de particules creuses à base d'au moins un polymère ou copolymère acrylique ou méthacrylique pour la préparation d'une lotion cosmétique et/ou dermatologique comprenant une phase aqueuse et une phase poudrée comportant au moins une poudre active, pour éviter le colmatage de la phase poudrée et conférer à la lotion de la douceur lors de l'application sur la peau, les muqueuses et/ou le cuir chevelu.

Dans le cadre de la présente invention, le mot « lotion » signifie que la composition est liquide et a donc une viscosité inférieure à 0,1 Pa.s (100 centipoises), de préférence inférieure à 0,06 Pa.s (60 centipoises), cette viscosité étant mesurée à 25°C avec un appareil Contraves Rheomat 108/ER. La lotion de l'invention comprend uniquement une phase aqueuse et une phase poudrée, et est donc exempte d'huiles.

La lotion selon l'invention est destinée à une application topique et contient donc un milieu physiologiquement acceptable, c'est-à-dire compatible avec la peau, les muqueuses et/ou le cuir chevelu.

La quantité de phase poudrée dans la lotion selon l'invention peut aller de 0,01 à 10 %, de préférence de 0,2 à 8 % en poids par rapport au poids total de la lotion.

La poudre active de la lotion selon l'invention peut être choisie parmi toutes les poudres habituellement utilisées dans les lotions poudrées et ayant notamment une activité de soin, de démaquillage, de nettoyage et/ou de coloration de la peau, des muqueuses et/ou du cuir chevelu, il peut s'agir par exemple de poudres matifiantes, nettoyantes, démaquillantes, colorantes, astringentes, anti-bactériennes et/ou protectrices (par exemple protection UV).

Comme poudre active, on peut citer par exemple le kaolin, l'oxyde de zinc, les poudres de polyéthylène, les poudres de polyamide et notamment les poudres de Nylon répertoriées sous le nom CTFA ("International Cosmetic Ingredient Dictionary and Handbook")de "Nylon 12" ou "Nylon 6" comme par exemple celles vendues sous la dénomination "ORGASOL" par la Société ATOCHEM, les poudres d'origine végétale comme les poudres de henné ou de santal, les oxydes de titane et les nanotitanes (ou nano-oxydes de titane), les oxydes de fer et nano-oxydes de fer, l'amidon, la poudre d'anhydride silicique. On peut aussi utiliser un mélange de ces poudres.

La quantité de poudre active dans la lotion selon l'invention va de préférence de 0,005 à 9,5 % et mieux de 0,1 à 5 % en poids par rapport au poids total de la lotion.

Les particules creuses utilisables pour la préparation de la lotion selon l'invention peuvent être réalisées à partir de monomères d'acide acrylique ou méthacrylique ou de monomères d'esters d'acide acrylique ou méthacrylique tels que l'acrylate ou le méthacrylate de méthyle, seuls ou en copolymérisation avec d'autres monomères à insaturation éthylénique, tels que le chlorure de vinylidène, l'acrylonitrile, le styrène et ses dérivés, le butadiène et ses dérivés et leurs mélanges. Les polymères constituant ces particules creuses peuvent être réticulés ou non.

La cavité interne des particules contient en principe un gaz qui peut être de l'air, de l'azote ou un hydrocarbure comme l'isobutane ou l'isopentane.

Comme particules creuses utilisables pour la préparation de la lotion de l'invention, on peut citer par exemple les particules de polyméthylméthacrylate, telles que celles commercialisées par la société Wackherr sous la dénomination "Covabead LH 85".

Les particules creuses peuvent être choisies aussi parmi les particules d'un copolymère expansé de chlorure de vinylidène, d'acrylonitrile et de méthacrylate de méthyle, et notamment les particules de terpolymère expansé de chlorure de vinylidène, d'acrylonitrile et de méthacrylate, vendues sous la marque EXPANCEL par la société Nobel Casco sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23. Les particules de terpolymère indiquées ci-dessus peuvent être sèches ou hydratées et peuvent être obtenues, par exemple, selon les procédés des brevets et demandes de brevets EP-A-056219, EP-A-348372, EP-A-486080, EP-A-320473, EP-A-112807 et US-A-3615972.

On peut aussi utiliser un mélange de ces différentes particules.

De façon avantageuse, les particules creuses de l'invention ont une granulométrie allant de 1 µm à 100 µm et encore mieux allant de 5 µm à 80 µm.

Selon un mode préféré de réalisation de l'invention, on utilise comme particules creuses les particules de polyméthylméthacrylate qui ont l'avantage de permettre une décantation plus rapide des phases aqueuse et poudrée après agitation.

Les particules creuses de la lotion selon l'invention sont présentes dans des concentrations allant de préférence de 0,005 à 9,5 % et mieux de 0,1 à 5 % en poids par rapport au poids total de la lotion.

Comme indiqué ci-dessus, on entend par "phase aqueuse" dans la lotion selon l'invention la phase liquide de la lotion par opposition à la phase poudrée. Cette phase aqueuse peut être entièrement constituée d'eau ou être hydroalcoolique, c'est-à-dire additionnée d'un ou de plusieurs solvants miscibles à l'eau, tels qu'un alcool(s) primaire(s).

La phase aqueuse de la lotion selon l'invention représente de 90 à 99,99 % et de préférence de 92 à 99,8 % en poids par rapport au poids total de la lotion. La phase aqueuse contient de préférence au moins 40 % et mieux au moins 50 % en poids d'eau par rapport au poids total de la lotion.

Comme alcool primaire, on peut utiliser dans la lotion de l'invention tout alcool aliphatique à chaîne linéaire ou ramifiée comportant de 1 à 6 atomes de carbone et de préférence de 2 à 4 atomes de carbone. Comme alcool primaire, on peut citer par exemple l'éthanol, le propanol et l'isopropanol. On utilise de préférence l'éthanol ou un mélange d'éthanol et d'un autre alcool primaire tel que l'isopropanol.

Quand la lotion contient un ou des alcools primaires, la quantité d'alcool(s) primaire(s) peut varier dans une large mesure selon le but escompté pour la lotion selon l'invention. Elle peut aller par exemple de 0,01 à 55 % et de préférence de 0,1 à 40 % en poids par rapport au poids total de la lotion.

La phase aqueuse peut comprendre aussi d'autres additifs habituellement utilisés dans ce type de lotion et notamment un ou plusieurs polyols tels que la glycérine, l'éthylène glycol, le propylène glycol, le butylène glycol, le dipropylène glycol, les polyglycérines comme la diglycérine, la triglycérine et la tétraglycérine, le sorbitol, les sucres et leurs mélanges.

La lotion selon l'invention peut contenir une quantité de polyol(s) allant par exemple de 0,01 à 20 % et de préférence de 0,1 à 10 % en poids par rapport au poids total de la lotion.

L'eau de la phase aqueuse peut être de l'eau pure ou déminéralisée. Toutefois, une partie de l'eau utilisée dans les compositions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut par exemple être choisie parmi l'eau de Vittel, les eaux du bassin de Vichy, l'eau d'Uriage, l'eau de La Roche Posay, l'eau de La Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-Les-Bains, l'eau de Lons-le-Saunier, l'eau des Eaux Bonnes, l'eau de Rochefort, l'eau de Saint Christau, l'eau des Fumades et l'eau de Tercis-les-bains, l'eau d'Uriage-les-Bains, l'eau d'Avene.

Les lotions de l'invention peuvent, en outre, contenir des adjuvants hydrosolubles utilisés dans le domaine cosmétique et/ou dermatologique, tels que les conservateurs, les antioxydants, les parfums, les filtres, les matières colorantes, les actifs hydrophiles. Comme actifs, on peut citer par exemple les vitamines telles que le panthénol, les dérivés des vitamines et notamment leurs esters, les agents antibactériens, les agents éclaircissants ou blanchissants, les hydratants, les agents kératolytiques tels que les alpha-hydroxyacides, les agents apaisants, les agents tonifiants tels que les extraits végétaux comme l'extrait de ruscus.

La lotion de l'invention peut constituer notamment une lotion de soin, de nettoyage, de démaquillage et/ou de coloration de la peau, des muqueuses et/ou du cuir chevelu.

L'invention a donc aussi pour objet l'utilisation cosmétique de la lotion telle que définie ci-dessus, pour le soin, le nettoyage, le démaquillage et/ou la coloration de la peau, des muqueuses et/ou du cuir chevelu.

La lotion de l'invention peut convenir aussi pour le traitement des peaux grasses.

L'invention a encore pour objet l'utilisation de la lotion telle que définie ci-dessus, pour la préparation d'une composition destinée au traitement des peaux grasses.

Les exemples qui suivent servent à illustrer l'invention sans toutefois présenter un caractère limitatif. Les quantités y sont indiquées en pourcentages en poids.

### Exemple 1

### Phase aqueuse

- Ethanol 15 %
- Glycérine 5 %
- Panthénol 1 %
- Extrait de ruscus 0,1 %
- Conservateurs qs
- Parfum qs
- Eau qsp 100 %

### Phase poudrée

- Kaolin 1 %
- Oxyde de Zinc 0,3 %
- Covabeads LH-85 1 %

La lotion est préparée en mélangeant les constituants suivants de la phase aqueuse :glycérine, panthénol, extrait de ruscus, conservateurs et eau, en y ajoutant l'éthanol dans lequel est solubilisé le parfum, puis en y incorporant les poudres une à une.

On obtient une lotion qui décante très facilement et dont la phase poudrée ne présente pas de colmatage. Elle est très douce à l'application et est apte à soigner la peau et notamment à l'hydrater et la tonifier.

### Exemple 2

### Phase aqueuse

- Ethanol 35 %
- Butylène glycol 5 %
- Conservateurs qs
- Parfum qs
- Eau qsp 100 %

### Phase poudrée

- Orgasol 0,3 %
- Expancel 551 DE 50 0,5 %

La lotion est préparée en mélangeant les constituants suivants de la phase aqueuse : butylène glycol, conservateurs et eau, en y ajoutant l'éthanol dans lequel est solubilisé le parfum, puis en y incorporant les poudres une à une.

On obtient une lotion qui décante facilement et dont la phase poudrée ne présente pas de colmatage. Elle permet un bon nettoyage de la peau tout en étant très douce à l'application.

## Revendications

1. Lotion cosmétique et/ou dermatologique comprenant une phase aqueuse et une phase poudrée comportant au moins une poudre active, caractérisée en ce que la phase poudrée comporte des particules creuses à base d'au moins un polymère ou copolymère acrylique ou méthacrylique, la phase aqueuse étant présente en une quantité allant de 90 à 99,99 % en poids par rapport au poids total de la lotion.

2. Lotion selon la revendication 1, caractérisée en ce que la poudre active est une poudre ayant une activité de soin, de démaquillage, de nettoyage et/ou de coloration de la peau, des muqueuses et/ou du cuir chevelu.

3. Lotion selon la revendication 1 ou 2, caractérisée en ce que la poudre active est choisie parmi les poudres matifiantes, nettoyantes, démaquillantes, colorantes, astringentes, antibactériennes et/ou protectrices.

4. Lotion selon l'une quelconque des revendications précédentes, caractérisée en ce que la poudre active est choisie parmi le kaolin, l'oxyde de zinc, les poudres de polyéthylène, les poudres de polyamide, les poudres d'origine végétale, les oxydes de titane et les nanotitanes, les oxydes de fer et nano-oxydes de fer, l'amidon, la poudre d'anhydride silicique et leurs mélanges.

5. Lotion selon l'une quelconque des revendications précédentes, caractérisée en ce qu'elle contient de 0,005 à 9,5 % en poids de poudre active par rapport au poids total de la lotion.

6. Lotion selon l'une quelconque des revendications précédentes, caractérisée en ce que les particules creuses sont réalisées à partir de monomères d'acide acrylique ou méthacrylique ou de monomères d'esters d'acide acrylique ou méthacrylique, seuls ou en copolymérisation avec d'autres monomères à insaturation éthylénique.

7. Lotion selon l'une quelconque des revendications précédentes, caractérisée en ce que les particules creuses sont choisies parmi les particules de polyméthylméthacrylate, les particules de terpolymère expansé de chlorure de vinylidène, d'acrylonitrile et de méthacrylate, et leurs mélanges.

8. Lotion selon l'une quelconque des revendications précédentes, caractérisée en ce que les particules creuses ont une granulométrie allant de 1 µm à 100 µm et de préférence allant de 5 µm à 80 µm.

9. Lotion selon l'une quelconque des revendications précédentes, caractérisée en ce qu'elle contient de 0,005 à 9,5 % en poids de particules creuses par rapport au poids total de la lotion.

10. Lotion selon l'une quelconque des revendications précédentes, caractérisée en ce que la phase aqueuse contient au moins 40 % en poids d'eau par rapport au poids total de la lotion.

11. Lotion selon l'une quelconque des revendications précédentes, caractérisée en ce que la phase aqueuse contient au moins un alcool primaire.

12. Lotion selon la revendication précédente, caractérisée en ce que l'alcool primaire est choisi parmi les alcools aliphatiques comportant de 2 à 4 atomes de carbone.

13. Lotion selon la revendication 11 ou 12, caractérisée en ce que la quantité d'alcool(s) primaire(s) va de 0,01 à 55 % en poids par rapport au poids total de la lotion.

14. Lotion selon l'une quelconque des revendications précédentes, caractérisée en ce que la phase aqueuse contient au moins un polyol.

15. Lotion selon la revendication précédente, caractérisée en ce que le polyol est choisi parmi la glycérine, l'éthylène glycol, le propylène glycol, le butylène glycol, le dipropylène glycol, les polyglycérines, le sorbitol, les sucres et leurs mélanges.

16. Lotion selon la revendication 14 ou 15, caractérisée en ce que la quantité de polyol(s) va de 0,01 à 20 % en poids par rapport au poids total de la lotion.

17. Lotion selon l'une quelconque des revendications précédentes, caractérisée en ce qu'elle constitue une lotion de soin, de nettoyage, de démaquillage et/ou de coloration de la peau, des muqueuses et/ou du cuir chevelu.

18. Utilisation cosmétique d'une lotion selon l'une quelconque des revendications précédentes pour le soin, le nettoyage, le démaquillage et/ou la coloration de la peau, des muqueuses et/ou du cuir chevelu.

19. Utilisation de la lotion selon l'une quelconque des revendications 1 à 17, pour la préparation d'une composition destinée au traitement des peaux grasses.

20. Utilisation de particules creuses à base d'au moins un polymère ou copolymère acrylique ou méthacrylique pour la préparation d'une lotion cosmétique et/ou dermatologique comprenant une phase aqueuse et une phase poudrée comportant au moins une poudre active, pour éviter le colmatage de la phase poudrée et conférer à la lotion de la douceur lors de l'application sur la peau, les muqueuses et/ou le cuir chevelu.

## Claims

1. Cosmetic and/or dermatological lotion comprising an aqueous phase and a powdered phase comprising at least one active powder, characterized in that the powdered phase comprises hollow particles based on at least one acrylic or methacrylic polymer or copolymer, the aqueous phase being present in an amount ranging from 90 to 99.99% by weight with respect to the total weight of the lotion.

2. Lotion according to Claim 1, characterized in that the active powder is a powder which is active in caring for, removing make-up from, cleaning and/or colouring the skin, mucous membranes and/or scalp.

3. Lotion according to Claim 1 or 2, characterized in that the active powder is chosen from mattifying, cleaning, make-up-removing, colouring, astringent, antibacterial and/or protective powders.

4. Lotion according to any one of the preceding claims, characterized in that the active powder is chosen from kaolin, zinc oxide, polyethylene powders, polyamide powders, powders of vegetable origin, titanium oxides and nanotitaniums, iron oxides and nano-scale iron oxides, starch, silicic acid anhydride powder and their mixtures.

5. Lotion according to any one of the preceding claims, characterized in that it comprises from 0.005 to 9.5% by weight of active powder with respect to the total weight of the lotion.

6. Lotion according to any one of the preceding claims, characterized in that the hollow particles are prepared from acrylic or methacrylic acid monomers or from acrylic or methacrylic acid ester monomers, alone or by copolymerization with other monomers possessing ethylenic unsaturation.

7. Lotion according to any one of the preceding claims, characterized in that the hollow particles are chosen from poly(methyl methacrylate) particles, particles of an expanded terpolymer of vinylidene chloride, of acrylonitrile and of methacrylate, and their mixtures.

8. Lotion according to any one of the preceding claims, characterized in that the hollow particles have a particle size ranging from 1 µm to 100 µm and preferably ranging from 5 µm to 80 µm.

9. Lotion according to any one of the preceding claims, characterized in that it comprises from 0.005 to 9.5% by weight of hollow particles with respect to the total weight of the lotion.

10. Lotion according to any one of the preceding claims, characterized in that the aqueous phase comprises at least 40% by weight of water with respect to the total weight of the lotion.

11. Lotion according to any one of the preceding claims, characterized in that the aqueous phase comprises at least one primary alcohol.

12. Lotion according to the preceding claim, characterized in that the primary alcohol is chosen from aliphatic alcohols comprising from 2 to 4 carbon atoms.

13. Lotion according to Claim 11 or 12, characterized in that the amount of primary alcohol(s) ranges from 0.01 to 55% by weight with respect to the total weight of the lotion.

14. Lotion according to any one of the preceding claims, characterized in that the aqueous phase comprises at least one polyol.

15. Lotion according to ' the preceding claim, characterized in that the polyol is chosen from glycerol, ethylene glycol, propylene glycol, butylene glycol, dipropylene glycol, polyglycerols, sorbitol, sugars and their mixtures.

16. Lotion according to Claim 14 or 15, characterized in that the amount of polyol(s) ranges from 0.01 to 20% by weight with respect to the total weight of the lotion.

17. Lotion according to any one of the preceding claims, characterized in that it constitutes a lotion for caring for, cleaning, removing make-up from and/or colouring the skin, mucous membranes and/or scalp.

18. Cosmetic use of a lotion according to any one of the preceding claims for caring for, cleaning, removing make-up from and/or colouring the skin, mucous membranes and/or scalp.

19. Use of the lotion according to any one of Claims 1 to 17 in the preparation of a composition intended for the treatment of greasy skin.

20. Use of hollow particles based on at least one acrylic or methacrylic polymer or copolymer in the preparation of a cosmetic and/or dermatological lotion comprising an aqueous phase and a powdered phase comprising at least one active powder, for preventing the powdered phase from becoming clogged and for conferring, on the lotion, softness during application to the skin, mucous membranes and/or scalp.

## Patentansprüche

1. Kosmetische und/oder dermatologische Lotion, die eine wäßrige Phase und eine Pulverphase mit mindestens einem pulverförmigen Wirkstoff aufweist, dadurch gekennzeichnet, daß die Pulverphase Hohlpartikel auf der Basis mindestens eines Acryl- oder Methacrylpolymers oder Acryl- oder Methacrylcopolymers enthält, wobei die wäßrige Phase in einem Mengenanteil von 90 bis 99,99 Gew.-%, bezogen auf das Gesamtgewicht der Lotion, vorliegt.

2. Lotion nach Anspruch 1, dadurch gekennzeichnet, daß der pulverförmige Wirkstoff wirksam zur Pflege, zur Reinigung, zum Abschminken und/oder zum Färben der Haut, der Schleimhäute und/oder der Kopfhaut eingesetzt werden kann.

3. Lotion nach Anspruch 1 oder 2, dadurch gekennzeichnet, daß der pulverförmige Wirkstoff unter den mattierenden, reinigenden, abschminkenden, färbenden, adstringierenden, antibakteriellen und/oder schützenden Pulvern ausgewählt ist.

4. Lotion nach einem der vorhergehenden Ansprüche, dadurch gekennzeichnet, daß der pulverförmige Wirkstoff unter Kaolin, Zinkoxid, Polyethylenpulvern, Polyamidpulvern, Pulvern pflanzlicher Herkunft, Titanoxiden und Nanotitanen, Eisenoxiden und Eisennanooxiden, Stärke, Siliciumdioxidpulver und deren Gemischen ausgewählt ist.

5. Lotion nach einem der vorhergehenden Ansprüche, dadurch gekennzeichnet, daß sie 0,005 bis 9,5 Gew.-% pulverförmigen Wirkstoff, bezogen auf das Gesamtgewicht der Lotion, enthält.

6. Lotion nach einem der vorhergehenden Ansprüche, dadurch gekennzeichnet, daß die Hohlpartikel aus Monomeren von Acrylsäure oder Methacrylsäure oder Monomeren von Acrylsäureestern oder Methacrylsäureestern alleine hergestellt oder mit anderen Monomeren mit ethylenischer Doppelbindung copolymerisiert werden.

7. Lotion nach einem der vorhergehenden Ansprüche, dadurch gekennzeichnet, daß die Hohlpartikel unter den Partikeln von Polymethylmethacrylat und den Partikeln eines expandierten Terpolymers von Vinylidenchlorid, Acrylnitril und Methacrylat oder deren Gemischen ausgewählt sind.

8. Lotion nach einem der vorhergehenden Ansprüche, dadurch gekennzeichnet, daß die Hohlpartikel eine Partikelgröße von 1 bis 100 µm und vorzugsweise 5 bis 80 µm aufweisen.

9. Lotion nach einem der vorhergehenden Ansprüche, dadurch gekennzeichnet, daß sie 0,005 bis 9,5 Gew.-% Hohlpartikel, bezogen auf das Gesamtgewicht der Lotion, enthält.

10. Lotion nach einem der vorhergehenden Ansprüche, dadurch gekennzeichnet, daß die wäßrige Phase mindestens 40 Gew.-% Wasser, bezogen auf das Gesamtgewicht der Lotion, enthält.

11. Lotion nach einem der vorhergehenden Ansprüche, dadurch gekennzeichnet, daß die wäßrige Phase mindestens einen primären Alkohol enthält.

12. Lotion nach dem vorhergehenden Anspruch, dadurch gekennzeichnet, daß der primäre Alkohol unter den aliphatischen Alkoholen mit 2 bis 4 Kohlenstoffatomen ausgewählt ist.

13. Lotion nach Anspruch 11 oder 12, dadurch gekennzeichnet, daß der Mengenanteil des oder der primären Alkohole 0,01 bis 55 Gew.-%, bezogen auf das Gesamtgewicht der Zusammensetzung, beträgt.

14. Lotion nach einem der vorhergehenden Ansprüche, dadurch gekennzeichnet, daß die wäßrige Phase mindestens ein Polyol enthält.

15. Lotion nach dem vorhergehenden Anspruch, dadurch gekennzeichnet, daß das Polyol unter Glycerin, Ethylenglykol, Propylenglykol, Butylenglykol, Dipropylenglykol, Polyglycerinen, Sorbit, Zuckern und deren Gemischen ausgewählt ist.

16. Lotion nach Anspruch 14 oder 15, dadurch gekennzeichnet, daß der Mengenanteil des Polyols oder der Polyole im Bereich von 0,01 bis 20 Gew.-%, bezogen auf das Gesamtgewicht der Lotion, liegt.

17. Lotion nach einem der vorhergehenden Ansprüche, dadurch gekennzeichnet, daß es sich um eine Lotion zur Pflege, zur Reinigung, zum Abschminken und/oder zum Färben der Haut, der Schleimhäute und/oder der Kopfhaut handelt.

18. Kosmetische Verwendung einer Lotion nach einem der vorhergehenden Ansprüche zur Pflege, zur Reinigung, zum Abschminken und/oder zum Färben der Haut, der Schleimhäute und/oder der Kopfhaut.

19. Verwendung einer Lotion nach einem der Ansprüche 1 bis 17 zur Herstellung einer Zusammensetzung, die für die Behandlung fettiger Haut vorgesehen ist.

20. Verwendung von Hohlpartikeln auf der Basis mindestens eines Acryl- oder Methacrylpolymers oder Acryl- oder Methacrylcopolymers zur Herstellung einer kosmetischen und/oder dermatologischen Lotion, die eine wäßrige Phase und eine Pulverphase mit mindestens einem pulverförmigen Wirkstoff aufweist, um das Verfestigen der Pulverphase zu verhindern und zu bewirken, daß sich die Lotion beim Auftragen auf die Haut, die Schleimhäute und/oder die Kopfhaut weich anfühlt.
